(19) Europäisches Patentamt
European Patent Office
Office européen des brevets

(11) Publication number: 0 365 176 A1

# (12) EUROPEAN PATENT APPLICATION

(21) Application number: 89310140.2

(22) Date of filing: 04.10.89

(51) Int. Cl.5: C07H 19/04 , A61K 31/70

(30) Priority: 04.10.88 JP 249083/88

(43) Date of publication of application: 25.04.90 Bulletin 90/17

(84) Designated Contracting States: AT BE CH DE ES FR GB IT LI LU NL SE

(71) Applicant: SUNTORY LIMITED
1-40 Dojimahama 2-chome Kita-ku
Osaka-shi Osaka(JP)

(72) Inventor: Honda, Tadashi
302, 1-4-13, Tanaka-cho Higashinada-ku
Kobe-shi Hyogo(JP)
Inventor: Kawazoe, Toshiji
12-17, Obiki-cho
Tatebayashi-shi Gunma(JP)
Inventor: Nozawa, Shigenori
5-6, Azanakane Taketoyo-cho
Chita-gun Aichi(JP)
Inventor: Nakanishi, Toshihiro
11-18 Nakatsu-cho
Ibaraki-shi Osaka(JP)

(74) Representative: Ford, Michael Frederick et al
MEWBURN ELLIS 2 Cursitor Street
London EC4A 1BQ(GB)

## (54) Ellipticine glycoside derivatives.

(57) A crystalline 2-α-L-arabinopyranosyl-9-hydroxy-5,11-dimethyl-6H-pyrido[4,3-b]carbazolium bromide•monohydrate (I) having an antitumor effect.

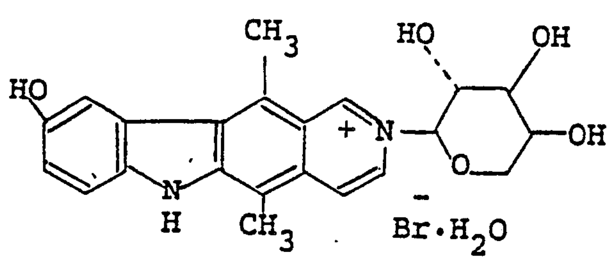

(I)

EP 0 365 176 A1

## ELLIPTICINE GLYCOSIDE DERIVATIVES

The present invention relates to a crystalline 2-α-L-arabinopyranosyl-9-hydroxy-5,11-dimethyl-6H-pyrido[4,3-b]carbazolium bromide•monohydrate having the formula [I]. The present compound has a potent anti-malignant tumor activity, and therefore is expected to be useful as an antitumor agent.

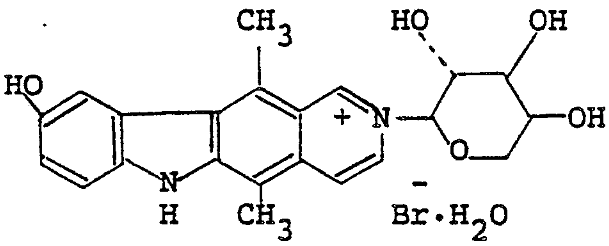

(I)

2. Description of the Related Art

Pyridocarbazole alkaloids such as ellipticine (5,11-dimethyl-6H-pyrido[4,3-b]carbazole (R=H in the following formula (A), 9-methoxyellipticine (R=$OCH_3$ in the formula (A)) and 9-hydroxyellipticine (R=OH in the formula (A)), are contained in Aspidospermina and Ochrosia leaves, which are a kind of alkaloid long-known in the art:

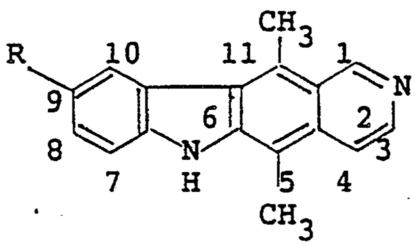

(A)

Recently, it was reported that 2-N-methyl-9-hydroxyellipticinium acetate of the formula (B):

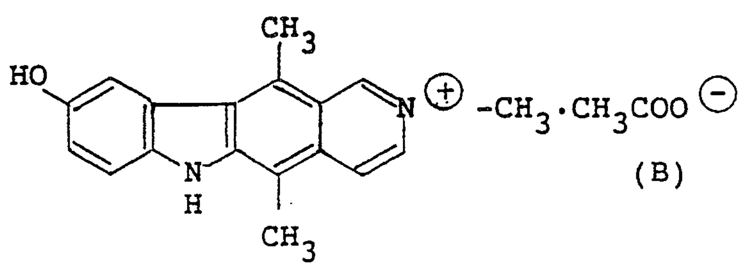

(B)

is effective against breast cancer [J. Rouessé et al, Bull. Cancer (Paris) vol. 68, pp. 437-441, 1981].

It has been also reported that ellipticine and 9-methoxyellipticine are effective against mouse leukemia L1210 and Sarcoma 180 (solid type) which are experimental animal tumors [R. W. Guthria et al, J. Medicinal Chemistry, Vol. 18 (7), pp. 775-760, 1975], and that 9-hydroxyellipticine has a 100 to 1000 fold higher activity against mouse leukemia L1210 than 9-methoxyellipticine (JP-B-58-35196, GB-A-1436 080).

As mentioned above, compounds having a Pyridocarbazole skeleton are useful as compounds having an antitumor activity, and a large number of synthesis methods have been studied, as can be seen from L. K. Dalion et al, Aust. J. Chem., Vol. 20, p.p. 2715-2727 (1967); A. H. Jackson et al, J. Chem. Soc. Perkin I, pp. 1698-1704 (1977); J. Y. Lallemand et al, Tetrahydron Letters, No. 15, 1261-1264 (1978), and EP-A-9445 among others.

Also, compounds having substituents introduced into the pyridocarbazole skeleton have been recognized to have an activity against mouse leukemia L1210 (U.S. Patent No. 4434290).

Nevertheless, ellipticine, 9-methoxyellipticine, and 9-hydroxyellipticine have not yet been practically applied as an antitumor agent; one reason for this is that these compounds are relatively non-soluble in water.

JP-A-58-222087 proposed the introduction of amino acid, oligopeptide, nucleotide or nucleoside at the 10-position of the skeleton, by oxidizing 2-N-alkyl-9-hydroxyellipticinium salt, but these compounds have not shown a favorable life prolongation effect against mouse leukemia L 1210.

The present inventors have synthesized compounds with various sugars having glycoside bonds at the 2-position of ellipticine derivatives (see Japanese Unexamined Patent Publication (Kokai) No. 61-37799), and made an intensive study of synthesis methods, main drug effects, behaviour in the body, and safety, etc. of these derivatives, and consequently, found that 2-α-L-arabinopyranosyl-9-hydroxy-5,11-dimethyl-6H-pyrido[4,3-b]carbazolium bromide is an excellent antitumor agent candidate substance.

Preferred objects of the present invention are to eliminate the above-mentioned disadvantages of the prior art and to provide a novel compound having a potent anti-malignant tumor activity, excellent stability, and a low hygroscopicitity.

Various optional and preferred features of the present invention will be apparent from the following description.

In accordance with the present invention, there is provided crystalline 2-α-L-arabinopyranosyl-9-hydroxy-5,11-dimethyl-6H-pyrido[4,3-b]carbazolium bromide•monohydrate, and methods of making it.

In accordance with the present invention, there is also provided a pharmaceutical composition comprising, as an essential component, crystalline 2-α-L-arabinopyranosyl-9-hydroxy-5,11-dimethyl-6H-pyrido[4,3-b]carbazolium bromide•monohydrate and a pharmaceutically acceptable carrier therefor.

The present invention will be better understood from the description set forth below with reference to the accompanying drawing of Fig. 1, which shows isothermal hygroscopic curves of 2-α-L-arabinopyranosyl-9-hydroxy-5,11-dimethyl-6H-pyrido[4,3-b]carbazolium bromide•monohydrate (B) and the anhydrous product compound (A).

2-α-L-arabinopyranosyl-9-hydroxy-5,11-dimethyl-6H-pyrido[4,3-b]carbazolium bromide is obtained by acid hydrolyzing 2-(2,3,4-tri-O-acetyl-α-L-arabinopyranosyl)-5,11-dimethyl-9-methoxy-6H-pyrido[4,3-b]-carbazolium] bromide synthesized by heating under reflux 9-methoxyellipticine and 2,3,4-tri-O-acetyl-β-L-arabinopyranosyl bromide in acetonitrile in the presence of triethylamine together with conc. aqueous hydrobromic acid, and recrystallizing same from hydrous alcohol. The crystals obtained, however, contain indefinite amounts of water and alcohol, and the anhydrous crystals obtained by drying under a reduced pressure at 80°C have a high hygroscopicity, as shown in Fig. 1. These drawbacks make it very difficult to set the standards of the original material, and to improve the storage stability, and accordingly, there is a demand for the development of a stable form and nonhygroscopic form.

The inventors found that 2-α-L-arabinopyranosyl-9-hydroxy-5,11-dimethyl-6H-pyrido[4,3-b]carbazolium bromide•monohydrate has a lower hygroscopicity than an anhydrous product of this compound, and has a good crystallinity.

The Ellipticine derivates embodying the present invention have remarkable anti-neoplastic or antitumor effects against mouse lymphoid leukemia L 1210 as shown in the Examples hereinbelow. It is considered that the present ellipticine derivatives are effective antineoplastic or antitumor agents, since the antineoplastic or antitumor activity of the present ellipticine derivatives is superior to that of Celiptium, which is clinically administered to patients with mammary cancer, used as a control.

When the present ellipticine derivatives are used as an antineoplastic or antitumor agent, they can be used in any form, for example, in the form of an injection such as intravenous, intramuscular, or subcutaneous injection, in the form of oral administration drugs such as tablets, granules, powder, or troches, or in the form of endermic drugs such as vaginal or rectal suppositories, or in the form of ointments.

In the practice of the formulation, any conventional and pharmaceutically acceptable ingredients including diluents, carriers, excipients, binders, and vehicles may be used. Pharmaceutically acceptable vehicles such as pharmaceutically acceptable liquid oil can be used as a suspending agent.

Examples of such excipients or carriers may include polyvinyl pyrrolidone, gum arabic, gelatin, sorbitol, cyclodextrins, tragacanth, magnesium stearate, talc, polyethylene glycol, polyvinyl alcohol, silica, lactose, crystalline cellulose, sugar, starch, calcium phosphate, vegetable oil, carboxymethyl cellulose calcium, sodium lauryl sulfate, water ethanol, glycerine, mannitol, syrup, and the like.

An anti-malignant tumor agent embodying the invention can contain the above-mentioned compound in an effective amount.

The effective amount of the present compound to be administered may be varied depending on the condition and the age of the patient to be treated, the administration route, the dosage form, the number of

times of administrations, and the like, but may be, for example, generally within the scope of from about 1.0 to 1000 mg, preferably from 10 to 800 mg, for a human adult per day.

## Examples

The present invention will now be further illustrated by, but is by no means limited to, the following Examples, wherein all parts and percentages are expressed on a weight basis unless otherwise noted.

### Example 1

Preparation of 2-α-L-arabinopyranosyl-9-hydroxy-5,11-dimethyl-6H-pyrido[4,3-b]carbazolium bromide•mono hydrate:

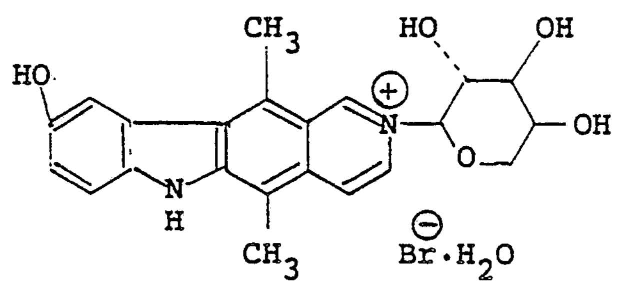

A 8.0 g (0.013 mole) amount of 2-(2,3,4-tri-O-acetyl-α-L-arabinopyranosyl)-5,11-dimethyl-9-methoxy-6H-pyrido[4,3-b]carbazolium bromide was added to 130 ml of 47% hydrobromic acid, the mixture was heated under reflux for 30 minutes, the reaction mixture thus obtained was ice-cooled, and precipitates were collected. The precipitates were dissolved in 100 ml of hot water, recrystallized by an addition of 100 ml of ethanol, and the crystals were washed three times and dried under a reduced pressure at 80° C and then in air and in the presence of phosphorus pentoxide, to give 6.0 g of the desired compound (yield 94%).

The compound obtained above was found to have the physical properties shown below. The purity was calculated by high performance chromatography (LC) measured under the following conditions.
Column: Shim-pack CLC-ODS 15 cm x 6 mmϕ
Mobile phase: Acetonitrile: 0.02 mole $NaH_2PO_4$-$H_3PO_4$ buffer = 1:5
Flow rate: 1.0 ml/min.
or Mobile phase
(Column: Shim-pack CLC-ODS 15cm x 6mmϕ):
acetonitrile:0.02 mole $NaH_2PO_4$-$H_3PO_4$ buffer = 1:4
Flow rate: 1.5 ml/min.

The compound obtained above was found to have the following physical and chemical properties.

m.p.: 250°C (decomposed)
$[\alpha]_D^{18°C}$: -230°C (c = 0.20, $H_2O$)

IR spectrum: (KBr, $cm^{-1}$) 3250, 1640, 1600, 1400, 1420, 1220, 1200, 1150, 1090, 1060, 810

UV spectrum: ethanol ($\lambda_{max}$ , nm) 227($\epsilon$14000) 249($\epsilon$23000) 267($\epsilon$25000) 282($\epsilon$22000) 324($\epsilon$52000)

Mass spectrum: (SIMS, m/Z) $395[C_{22}H_{23}H_{2}O_{5}]^{+}$

$^{1}$HNMR spectrum: ($DMSO-d_5$, $\delta$ppm, proton chemical shift of $CD_2HSOCD_3$ ($\delta$2.50) used as the internal standard) 2.85(3H,s) 3.65(1H,m) 3.90(3H,m) 4.08(1H,d) 5.12(1H,d) 5.24(1H,d) 5.61(1H,d) 5,74(1H,d,J=8.5Hz, 1-H) 7.18(1H,dd,J=2Hz,9Hz) 7.55 (1H,d,J=9Hz) 7.86(1H,d,J=2Hz) 8.51(2H,ABq) 9,45(1H,brs) 10.03 (1H,s) 12.00(1H,brs)

Elemental analysis: $C_{22}H_{23}N_{2}O_{5}Br \cdot H_{2}O$
values
Calcd(C, H, N): 53.55, 5.07, 5,68
Found(C, H, N): 53.43, 4.73, 5.64

X-ray analysis
Measurement conditions

- X-ray tube (counter-electrode) Cu
- Tube voltage 40 kV
- Tube current 30 mA
- Scanning speed 4°/min
- Full scale 5000 cps
- chart speed 40 mm/min
- Time const 0.5 sec
- Scattering slit 1°
- Light-receiving slit 0.3 mm
- Dispersing slit 1°

Measured data:

| d (Å) | Relative strength | | |
|---|---|---|---|
| | | 4.40 | Weak |
| 9.3 | Moderate | 4.20 | Very weak |
| 7.1 | Weak | 3.93 | Very weak |
| 6.3 | Very weak | 3.84 | Moderate |
| 5.30 | Moderate | 3.71 | Weak |
| 4.96 | Very Weak | 3.53 | Very Weak |
| 4.84 | Weak | 3.45 | Very strong |
| 4.63 | Weak | 3.37 | Weak |
| d (Å) | Relative strength | 2.76 | Weak |
| 3.24 | Weak | 2.64 | Very Weak |
| 3.16 | Moderate | 2.55 | Weak |
| 2.98 | Weak | 2.46 | Weak |

The above-mentioned data shows the d-value of the strongest line (note 2.4 Å or higher) and the relative line strength estimated by the naked eye.

Example 2

The difference between 2-α-L-arabinopyranosyl-9-hydroxy-5,11-dimethyl-6H-pyrido[4,3-b]carbazolium bromide•monohydrate and the anhydrous product of the compound was confirmed by the isothermal hygroscopic curve. The results are shown in Fig. 1.

Reference Example

Preparation of 2-(2,3,4-tri-O-acetyl-α-L-arabinopyranosyl)-5,11-dimethyl-9-methoxy-6H-pyrido[4,3-b]-carbazolium bromide:

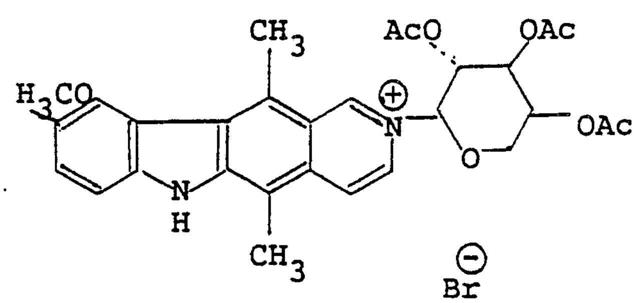

wherein, Ac represents an acetyl group.

An 8.28 g amount (0.03 mole) of 9-methoxyellipticine, 20.34 g (0.06 mole) of 2,3,4-tri-O-acetyl-L-arabinopyranosyl bromide, and 4.2 ml (0.03 mole) of triethylamine were suspended in 300 ml of acetonitrile, followed by heating under reflux for 5 minutes.

The reaction mixture thus obtained was quenched in ice-water, and the colloidal precipitates formed were collected. The precipitates were washed 3 times with chloroform-ether (1:4), and the precipitates (α:β = 100:1.1) were recrystallized by acetonitrile (880 ml) to obtain 7.56 g of the desired compound (yield 41%). The recrystallization mother liquor was concentrated and recrystallized to give an additional 3.53 g of the desired compound (yield 19%). The obtained compound was found to have the physical properties shown below.

The ratio of α to β was calculated from the area ratio of high performance liquid chromatography (HPLC) measured under the following conditions:
Column: Nova-pack C-18, 15 cm x 3.9 mmϕ

Mobile phase: 0.1 mole ammonium acetate-acetic acid (pH 3 buffer:methanol = 2:1)
Flow rate: 1.5ml/min.
Detection: 318 nm (UV detector)

The compound thus obtained was found to have the following physical properties.

Properties: amorphous crystals
$[\alpha]_D^{18°C}$: -46° (c = 0.16, $H_2O$)

| | |
|---|---|
| IR spectrum: (KBr, $cm^{-1}$) | 1750, 1640, 1590, 1480, 1420, 1370, 1300, 1240, 1220, 1120, 1090, 1060, 1030, 960, 930, 810 |
| UV spectrum: ethanol ($\lambda_{max}$, nm) | 228($\epsilon$15000) 249($\epsilon$24000) 256($\epsilon$24000) 268($\epsilon$24000) 282($\epsilon$21000) 325($\epsilon$52000) |
| Mass spectrum: (SIMS, m/Z) | 535$[C_{29}H_{31}H_2O_8]^+$ |
| $^1$HNMR spectrum: ($CDCl_3$, $\delta$ppm, TMS used as the internal standard) | 1.68(3H,s) 1.98(3H,s) 2.03(3H,s) 2.32(3H,s) 2.46(3H,s) 3.94(3H,s) 4.41(1H,dd, J=13Hz,1.8Hz) 4.65(1H,d,J=13Hz) 5.50(2H,m) 5.61(1H,brs) 7.06(1H,brs) 7.20(1H, d,J=9Hz,1′-H) 7.32(1H,dd,J=2Hz, 7Hz) 7.61(1H,d,J=7Hz) 8.12(1H,d, J=9Hz) 8.17(1H,d,J=9Hz) 10.18 (1H,s) 11.94(1H,brs) |

## Example 3

The antitumor effect of 2-α-L-arabinopyranosyl-9-hydroxy-5,11-dimethyl-6H-pyrido[4,3-b]carbazolium bromide•monohydrate was confirmed by using the experimental tumor L1210 of a mouse.

i) Animals used:
BDF$_1$ mouse, female, 6 weeks old (average weight 17 - 18 g), 6 mice per group;

ii) Tumor species used:
L1210 (mouse leukemia cells), $10^5$ cells/mouse, intraperitoneally inoculated (i.p.);

iii) Sample administration method:
L1210 was inoculated into a BDF1 mouse, and after 24 hours, administered intraperitoneally once per day continuously for 5 days;

iv) Evaluation method:
Effectiveness is shown by prolongation of average survival time compared with control group (ILS%).

$$ILS\% = \left( \frac{\text{Average survival days of administered group}}{\text{Average survival days of control group}} - 1 \right) \times 100$$

The results are as shown in Table 2, and from these results, it is clear that the present compound has a remarkable antitumor effect against mouse leukemia L1210.

The 80 days life prolongation shows that the number indicated of mice were alive 80 days after administration of the mouse leukemia L1210.

| Dose (mg/kg) | Toxicity | ILS % | 80 days survival |
|---|---|---|---|
| 5 | 0/6 | 73 | 0/6 |
| 10 | 0/6 | 116 | 0/6 |
| 20 | 0/6 | >393 | 2/6 |
| 40 | 0/6 | >967 | 6/6 |

## Claims

1. A crystalline 2-α-L-arabinopyranosyl-9-hydroxy-5,11-dimethyl-6Hpyrido[4,3-b]carbazolium bromide•monohydrate.

2. A crystalline 2-α-L-arabinopyranosyl-9-hydroxy-5,11-dimethyl-6H-pyrido[4,3-b]carbazolium bromide•monohydrate as claimed in claim 1, wherein lattice distance (d-values greater than 2,4 angstrom (Å)) and relative strengths of respective diffraction lines are as shown below in powder X-ray diffraction using copper as the counter-electrode:

| d (Å) | Relative Strength |
|---|---|
| 9.29 | Moderate |
| 7.15 | Weak |
| 6.35 | Very weak |
| 5.30 | Moderate |
| 4.96 | Very weak |
| 4.84 | Weak |
| 4.63 | Weak |
| 4.40 | Weak |
| 4.20 | Very weak |
| 3.93 | Very weak |
| 3.84 | Moderate |
| 3.71 | Weak |
| 3.53 | Very weak |
| 3.45 | Very strong |
| 3.37 | Weak |
| 3.24 | Weak |
| 3.16 | Moderate |
| 2.98 | Weak |
| 2.76 | Weak |
| 2.64 | Very weak |
| 2.55 | Weak |
| 2.46 | Weak |

3. A pharmaceutical composition comprising, as an essential component, 2-α-L-arabinopyranosyl-9-hydroxy-5,11-dimethyl-6H-pyrido[4,3- b]carbazolium bromide•monohydrate in crystalline form and a pharmaceutical acceptable carrier therefor.

4. A process comprising the production of the compound of claim 1 or claim 2.

5. The compound of claim 1 or claim 2, for use as an antitumour agent.

6. Use, in the manufacture of a medicament for the treatment of tumours, of 2-α-L-arabinopyranosyl-9-hydroxy-5,11-dimethyl-6H pyrido[4,3-b]carbazolium bromide.

Fig. 1

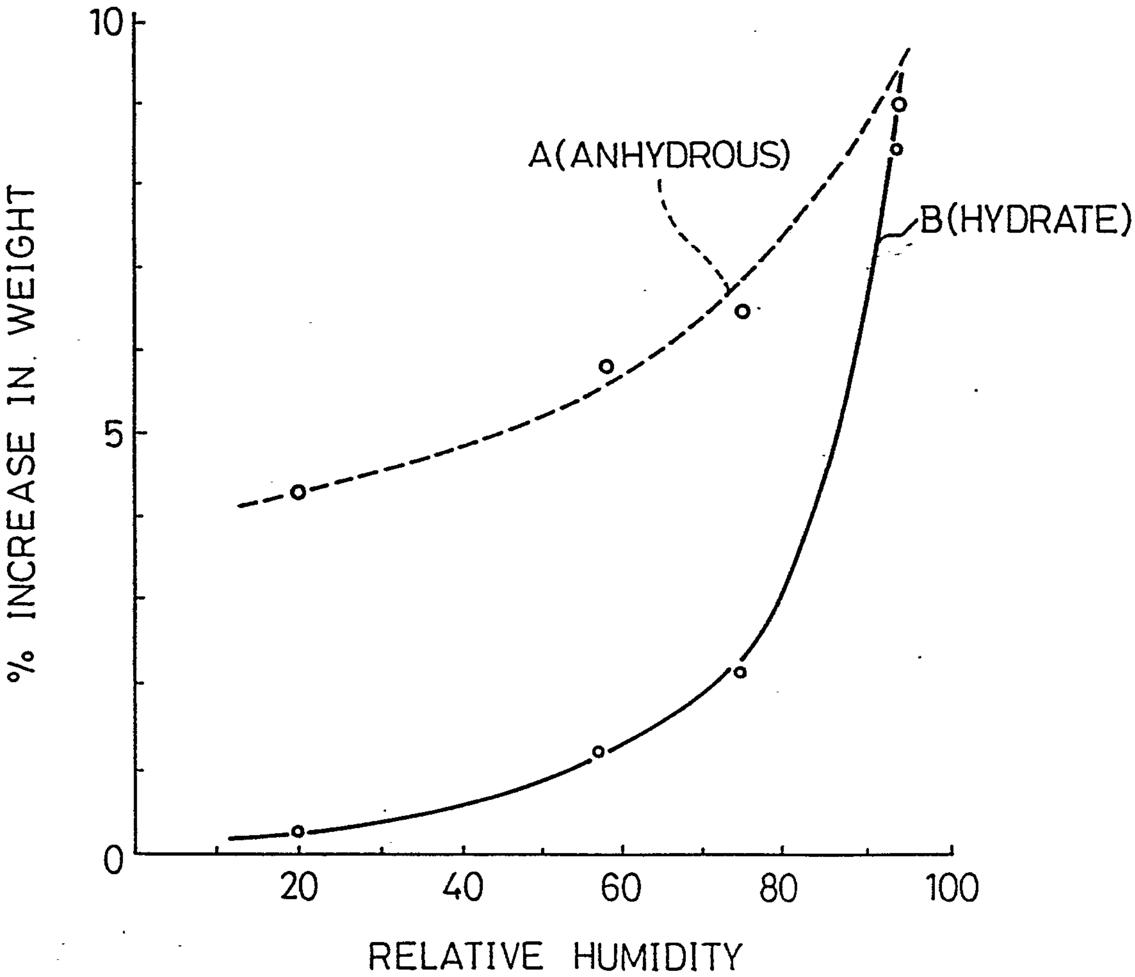
10
5
0
A(ANHYDROUS)
B(HYDRATE)
% INCREASE IN WEIGHT
20
40
60
80
100
RELATIVE HUMIDITY

European Patent Office

# EUROPEAN SEARCH REPORT

Application Number

EP 89 31 0140

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 173 462 (SUNTORY LTD)<br>* Abstract; page 48, example 85 *<br>--- | 1,3-6 | C 07 H 19/04<br>A 61 K 31/70 |
| X | CHEMICAL ABSTRACTS, vol. 111, no. 1, 3rd July 1989, page 750, abstract no. 7660j, Columbus, Ohio, US; & JP-A-63 215 690 (SUNTORY LTD) 08-09-1988<br>* Abstract *<br>--- | 1,3-6 | |
| X | J. MED. CHEM., vol. 31, no. 7, July 1988, pages 1295-1305, American Chemical Society; T. HONDA et al.: "Synthesis and antitumor activity of quaternary ellipticine glycosides, a series of novel and highly active antitumor agents"<br>* Abstract; page 1298, compound 41b; page 1303, column 1, lines 15-30 *<br>----- | 1,3-6 | |
| | | | TECHNICAL FIELDS SEARCHED (Int. Cl.5) |
| | | | C 07 H 19/00<br>A 61 K 31/00 |

The present search report has been drawn up for all claims

| Place of search | Date of completion of the search | Examiner |
|---|---|---|
| THE HAGUE | 10-01-1990 | SCOTT J.R.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)